(19) Europäisches Patentamt
European Patent Office
Office européen des brevets

(11) Numéro de publication: **0 436 414 A1**

(12) **DEMANDE DE BREVET EUROPEEN**

(21) Numéro de dépôt: **90403405.5**

(51) Int. Cl.5: **C07C 213/02**, C07C 215/10

(22) Date de dépôt: **30.11.90**

(30) Priorité: **03.01.90 FR 9000017**

(43) Date de publication de la demande: **10.07.91 Bulletin 91/28**

(84) Etats contractants désignés: **BE CH DE FR GB IT LI LU NL**

(71) Demandeur: **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07(FR)**

(72) Inventeur: **Rignon, Maurice**
**Les Jardins de Simplecour, Cidex 2029**
**F-71640 Givry(FR)**
Inventeur: **Benattar, André**
**La Tourette, La Verdarie**
**F-81100 Castres(FR)**
Inventeur: **Le Nard, Gilles**
**37, rue des Tuileries, Chatenoy-en-Bresse**
**F-71380 Saint Marcel(FR)**
Inventeur: **Malafosse, Jean**
**Chemin des Montagnes, Les Côtes**
**F-38360 Sassenage(FR)**

(74) Mandataire: **Le Moenner, Gabriel et al**
**L'AIR LIQUIDE, Société Anonyme pour l'étude et l'exploitation des procédés Georges Claude 75, Quai d'Orsay**
**F-75321 Paris Cédex 07(FR)**

(54) **Procédé de préparation de l'amino-2-propanediol-1,3 et de ses sels.**

(57) La présente invention concerne un procédé de préparation de l'amino-2-propanediol-1,3 (sérinol) ou de ses sels, dans lequel on réalise au moins les étapes suivantes :

a) on réalise une formylation par réaction du formaldéhyde et du nitrométhane en milieu aqueux alcalin;
b) on acidifie la solution obtenue;
c) on réalise l'électroréduction cathodique de la solution acidifiée; et
d) on sépare l'amino-2-propanediol-1,3 ou son sel de la solution cathodique.

On peut purifier la solution cathodique par électro-électrodialyse, ce qui conduit à une solution aqueuse pure de sérinol.

EP 0 436 414 A1

## "PROCEDE DE PREPARATION DE L'AMINO-2-PROPANEDIOL-1,3 ET DE SES SELS"

La présente invention concerne un procédé de préparation de l'amino-2-propanediol-1,3, également appelé sérinol.

Le sérinol est un aminoalcool intermédiaire de synthèse utilisé notamment pour la préparation d'agents de contraste en angiographie et en myelographie, ces agents étant particulièrement utiles à cause de leur très basse toxicité et de leur bonne compatibilité.

Il est connu de préparer le sérinol par hydrogénation catalytique du sel de sodium du nitropropanediol. Ces procédés connus ont cependant les inconvénients de l'hydrogénation catalytique des dérivés nitrés, et en particulier la forte exothermie de la réaction qui oblige à utiliser des autoclaves spécialement conçus ou à diluer fortement le milieu réactionnel. Ces techniques de préparation sont en particulier décrites dans le brevet SCHERING US-A-4 221 740 et le brevet européen DYNAMIT NOBEL EP-A-071 037. A cause de sa sélectivité faible, le rendement de l'hydrogénation reste inférieur à 80% par rapport au nitronate initial. De plus, la purification de produit obtenu nécessite la mise en oeuvre de procédés de séparation complexes.

Il est connu de réaliser l'électro-réduction de dérivés nitrés aliphatiques, en particulier par la demande de brevet française FR-A-2 614 044. Cette demande de brevet concerne notamment la préparation d'aminoalcools tels que l'amino-2-méthyl-2-propanol-1, l'amino-2-méthyl-2-propanediol-1,3, l'amino-2-butanol-1, l'amino-2-éthyl-2-propanediol-1,3 et le tris(hydroxyméthyl)aminométhane, les produits de départ étant les nitroalcools correspondants.

Le nitroalcool correspondant au sérinol, le nitro-2-propanediol-1,3 comporte un atome d'hydrogène sur le même carbone que le groupement nitro; ceci rend la molécule assez fragile.

Compte tenu des applications ultérieures des produits synthétisés à partir du sérinol, il est important de réduire au minimum les produits de dégradation, et il est donc apparu très avantageux de préparer du sérinol à partir d'un sel du nitropropanediol plus stable que le nitropropanediol lui-même, et sans les inconvénients liés à l'hydrogénation catalytique de ces sels.

La présente invention concerne un procédé de préparation de l'amino-2-propanediol-1,3 à partir de nitrométhane et de formaldéhyde.

Le procédé de préparation de l'amino-2-propanediol-1,3, conforme à l'invention, comprend essentiellement les étapes suivantes

a) on réalise une formylation par réaction du formaldéhyde et du nitrométhane en milieu aqueux alcalin;

b) on acidifie la solution;

c) on procède à l'électroréduction cathodique de la solution acidifiée;

d) on sépare l'amino-2-propanediol-1,3 ou son sel de la solution cathodique.

Du fait de la sélectivité de la réaction électrochimique, et comme il n'est pas nécessaire de séparer le sel intermédiaire du type nitronate, à la fin de l'étape a), la mise en oeuvre du procédé selon l'invention est particulièrement simple et le rendement par rapport à la nitroparaffine de départ est excellent.

On réalise dans l'étape a) la formylation du nitrométhane en mettant en oeuvre la réaction

$$CH_3NO_2 + 2HCHO + MOH \longrightarrow CH_2OH - \underset{\displaystyle NO_2}{\overset{\displaystyle M^{\oplus}}{C^{\ominus}}} - CH_2OH + H_2O$$

L'aldéhyde formique peut être utilisé sous diverses formes, par exemple une solution aqueuse à 30% environ, ou la forme polymérisée connue sous le nom de polyoxyméthylène ou le paraformaldéhyde.

Le milieu aqueux alcalin utilisé dans l'étape a) peut être réalisé par apport d'une base forte minérale, $M^+$ pouvant représenter $Na^+$, $K^+$, $Li^+$ ou $NH_4^+$, par exemple. On obtient ainsi une solution de nitronate alcalin.

La concentration en $OH^-$ dans le milieu alcalin utilisé dans l'étape a) lors de la préparation du sel de métal alcalin ou d'ammonium de nitropropanediol est, selon l'invention, de préférence supérieure de 20% à la concentration stoechiométrique, et plus particulièrement supérieure de 5% à la concentration correspondant à la stoechiométrie.

Lors de l'addition de l'agent alcalin, la température du milieu réactionnel est de préférence contrôlée et maintenue inférieure à une température de 50 °C, et de préférence inférieure à une température d'environ 35 °C.

Lorsque le polyoxyméthylène est utilisé, la dépolymérisation a lieu d'abord; le produit pulvérulent est

mis en présence d'une petite quantité d'agent alcalin et d'eau (environ 5% de la quantité totale nécessaire), puis le nitrométhane est ajouté lentement. Tout en maintenant le milieu réactionnel sous bonne agitation et à une température modérée, on additionne lentement le reste de l'agent alcalin de telle façon que la quantité totale ajoutée soit en léger excès sur la stoechiométrie de la réaction.

Dans la seconde étape b), selon l'invention, on prépare une solution apte à être électrolysée, sans séparer le sel de métal alcalin ou d'ammonium du nitropropanediol formé en a).

Pour l'acidification, on utilise des solutions d'acides aqueuses concentrées et en particulier comme solution d'acide minéral, on peut utiliser par exemple une solution aqueuse à 33% d'acide chlorhydrique ou une solution aqueuse à 50% d'acide sulfurique. Pendant cette étape, il est préférable de maintenir la température du milieu, par exemple à l'aide d'un bain de glace ou de tout autre moyen susceptible de refroidir le réacteur, à une température inférieure à la température ambiante, et de préférence inférieure à $10^{\circ}$ c.

Les conditions de température, d'agitation et de pH sont importantes pour éviter des réactions parasites qui produisent des produits carbonés indésirables qui viendraient perturber la réaction électrochimique et diminuer son rendement.

L'agitation est de préférence vigoureuse, l'addition de l'acide lui-même étant de préférence effectuée dans une zone elle-même très bien agitée, et les vitesses d'addition des réactifs alcalins et acides sont réglées de telle façon que le pH reste en tout point du milieu compris entre 5 et 7.

Comme acide, on peut utiliser tout acide minéral courant, et en particulier l'acide méthane-sulfonique, benzène-sulfonique, toluène-sulfonique, chlorhydrique, bromhydrique, fluorhydrique, nitrique, phosphorique ou sulfurique.

Selon l'acide utilisé, en opérant à la température de cristallisation du sel alcalin correspondant à la neutralisation de la base par l'acide, on peut, dans une variante du procédé, séparer ce sel qui cristallise. La séparation peut être effectuée par tout moyen usuel comme la centrifugation, la filtration ou l'essorage centrifuge.

Ainsi, si on utilise NaOH puis $H_2SO_4$, en refroidissant le mélange obtenu en b) à un pH voisin de 5 et à une température d'environ 0 à $5^{\circ}$ C, $Na_2SO_4.10H_2O$ cristallise et précipite en cristaux qui peuvent être très facilement séparés par filtration ou essorage centrifuge.

La demanderesse a mis en évidence que la quantité d'acide utilisée à l'étape b) optimale pour l'électrolyse peut être définie par la formule suivante :

$$\text{nombre moles } H^{+} = \text{nombre moles } OH^{-} + (\text{nombre moles } R\text{-}NO_2) \times n$$

n étant de préférence compris entre 1 et 1,5, et en particulier égal à environ 1,3.

Cette quantité d'acide peut être ajoutée en une ou plusieurs fois, selon le temps de stockage de la solution avant électrolyse et selon qu'on se propose ou non de séparer le sel minéral de neutralisation.

Ainsi, comme il est important que durant la réaction de l'acidification ou durant un stockage éventuel de la solution acidifiée, avant électrolyse, le pH soit maintenu entre 5 et 7, on utilise seulement une partie de la quantité d'acide définie ci-dessus, le complément étant ajouté au moment de l'électrolyse pour laquelle le pH est d'environ 1.

La solution propre à être électrolysée a une concentration initiale d'acide telle que $H^{+}/C_3H_7O_2\text{-}NO_2$ est compris entre 1 et 1,5, de préférence égal à environ 1,3.

Dans l'étape suivante c), on réalise la réduction électrochimique de la solution acide électrolysable décrite ci-dessus, avec ou sans séparation du sel de neutralisation, sur une cathode bimétallique constituée d'un métal support et d'un élément actif.

La solution issue de l'étape b) est placée dans le circuit cathodique qui peut comprendre, outre la cellule, une pompe de recirculation, un échangeur thermique et un bac tampon.

Le métal support est choisi parmi les métaux bon conducteurs électriques, inattaquables dans le milieu réactionnel, et l'élément actif change d'état selon le potentiel du métal support en étant soit sous forme de cation en solution dans le catholyte, soit sous forme de dépôt métallique sur le métal support, de telle sorte que ces transformations obtenues simultanément aux réactions successives de réduction du dérivé nitré, entraînent à chaque opération un renouvellement complet de la surface électroactive à forte surtension d'hydrogène. Comme exemples d'élément actif, on peut citer le zinc, le cadmium ou l'étain. Pour le matériau-support, le choix est large. Conviennent, notamment, le plomb, l'acier inoxydable, le nickel et le cuivre, et le matériau préféré est le cuivre. Un tel procédé de réduction électrochimique est plus particulièrement décrit dans la demande de brevet FR-A-2 614 044 au nom de la demanderesse.

La réduction est effectuée en opération discontinue; au début de la première opération, on ajoute au catholyte une petite quantité de sel soluble de $Zn^{++}$, $Cd^{++}$ ou $Sn^{++}$ ou un mélange de sels de ces

cations. Quand la réduction est terminée, ce qui apparaît par dosage potentiométrique ou par détection par la liqueur de Fehling , la quasi-totalité des cations ajoutés se trouve à l'état de dépôt métallique sur la cathode. La détection à la liqueur de Fehling permet de déceler l'absence ou la présence d'hydroxylamino-2-propanediol-1,3.

Dans l'opération suivante, au début de la réduction, le dépôt se dissout dans le catholyte pour se déposer à nouveau en fin d'opération et recréer une surface cathodique neuve et très active. On peut ainsi enchaîner sur la même cathode de très nombreuses opérations de réduction, en assurant périodiquement un apport de cations actifs pour compenser la petite quantité qui est entraînée dans le catholyte réduit.

La quantité d'élements actifs sous forme cations, rapportés à la surface de la cathode, peut être comprise entre 1 et 10 mmoles.$dm^{-2}$, et de préférence de 2 à 6 mmoles.$dm^{-2}$.

On peut utiliser des anodes réalisées en titane platiné ou revêtu d'oxyde d'iridium, ce choix n'étant pas essentiel selon l'invention. De même, la solution anodique peut être une solution d'acide sulfurique ou toute autre solution anodique convenable.

Le système Cu-Zn (ou Cu-Cd ou Cu-Sn) est particulièrement intéressant, car sur une cathode en cuivre, le dépôt de Zn (ou Cd ou Sn) est préparé in situ simultanément à la réduction du dérivé nitré, et la quantité de $Zn^{++}$ (ou $Cd^{++}$ ou $Sn^{++}$) nécessaire peut être obtenue, soit par addition de sels minéraux de zinc, (ou de cadmium ou d'étain), soit par dissolution du dépôt de zinc (ou de cadmium ou d'étain) sur la lame de cuivre provenant d'une opération antérieure de réduction. Le choix du zinc présente l'avantage d'une toxicité très faible.

La température de la solution cathodique pendant l'électrolyse est de préférence comprise entre 20 et 100° C.

Lors de la réalisation de l'électro-réduction cathodique, la demanderesse a pu mettre en évidence que le rendement optimal était atteint en menant la réduction en deux phases successives : dans une première phase, jusqu'à ce que soit consommé une quantité de courant représentant entre environ la moitié et les deux tiers du courant efficace nécessaire, la température du catholyte est maintenue inférieure à 35° C, de préférence inférieure à environ 30° C, et dans une seconde phase, sa température est maintenue supérieure à 50° C, de préférence supérieure à environ 60° C.

A la fin de la réduction, selon l'invention, dans l'étape d), le sérinol ou l'un de ses sels est récupéré à partir de la solution cathodique, par tout procédé usuel. Ainsi, après neutralisation des protons libres, on peut évaporer à sec sous pression réduite. Le résidu est alors repris par un alcool à bas poids moléculaire, par exemple le méthanol, l'éthanol, un propanol ou un butanol. Une partie du sel de neutralisation, par exemple le chlorure de sodium, insoluble, peut être éliminée par filtration. Le solvant est éliminé lui-même sous pression réduite et le résidu est le sel attendu de bonne pureté. Pour améliorer encore la pureté, on peut recristalliser en utilisant un solvant convenable, du type alcool de bas poids moléculaire (C1 à C6), solvant chloré comme $CH_2Cl_2$, $CHCl_3$, $CCl_4$ ou $C_2H_4Cl_2$, ester comme acétate d'éthyle ou acétate d'isopropyle, ou leurs mélanges.

Ainsi, si l'acide utilisé est HCl, on obtient après électrolyse, une solution aqueuse pure de chlorhydrate de sérinol; par évaporation de l'eau et éventuellement recristallisation, on obtient ce chlorhydrate sous forme commercialisable. Pour obtenir d'autres sels du sérinol, le procédé est mis en oeuvre de façon analogue, avec un autre acide. Pour obtenir le sérinol lui-même, on procède par neutralisation puis séparation, de façon usuelle.

Mais, comme il a été décrit plus haut, dans une variante du procédé, on peut séparer entre les étapes b) et c), le sel de neutralisation obtenu et, de cette manière, on élimine un grande majorité du cation alcalin (par exemple $Na^+$). Cette variante présente l'avantage d'un rendement électrique de l'électrolyse amélioré. De plus, pour obtenir du sérinol sous forme libre, cette solution électrolysée peut être soumise au traitement d'électro-électrodialyse qui est un moyen simple pour éliminer les anions (par exemple $SO_4^{2-}$) et obtenir une solution quasi pure de l'amino-alcool. L'électro-électrodialyse peut être mise en oeuvre comme il est décrit dans la demande de brevet FR-A-2 577 242 au nom de la demanderesse.

Il s'agit d'un traitement de la solution aqueuse contenant les ions $C_3H_7O_2NH_3^+$, $H^+$ et $A^-$ ($A^-$ anion de l'acide utilisé à l'étape b)), ainsi qu'une quantité mineure d'ions $M^+$, par un champ électrique continu dans le compartiment cathodique d'une cellule électrochimique munie d'un diaphragme comportant une membrane perméable aux anions. On obtient dans le compartiment cathodique une solution aqueuse contenant le sérinol et une infime quantité de sel MA, et dans le compartiment anodique une solution aqueuse diluée de l'acide HA. Pour obtenir le sérinol sous forme très pure, on peut alors, parmi les procédés usuels, choisir l'évaporation rapide en couche mince sous vide poussé, ou encore, après évaporation de l'eau, la distillation sous pression réduite.

D'autres caractéristiques et avantages de l'invention apparaîtront à la lecture des exemples de réalisation ci-dessous.

EXEMPLE 1

Préparation du sel de sodium du nitro-2-propanediol-1,3.

Dans un réacteur ouvert muni d'un dispositif d'agitation à turbine multipale capable de malaxer et d'homogénéiser des pâtes visqueuses, on introduit 600 mmoles de polyoxyméthylène (HCHO)3 sous forme pulvérulente. On ajoute 20 g d'eau puis 6 mmoles de NaOH sous forme d'une solution aqueuse 10 N.

Le polyoxyméthylène passe en solution pour une partie, et la réaction de dépolymérisation de l'aldéhyde formique étant endothermique, la température du milieu réactionnel décroît, et ce jusqu'à environ 10 à 15° C.

On ajoute alors lentement 300 mmoles de $NO_2$-$CH_3$. La réaction étant exothermique, la température s'élève rapidement à 35° C, et on maintient cette température pendant la durée de l'addition, soit environ 15 minutes. On continue d'agiter le milieu réactionnel, qui est alors une solution limpide, pendant environ 15 minutes.

On ajoute ensuite, en 15 minutes, une solution de soude 10 N (309 mmoles); la réaction étant exothermique, on refroidit pour maintenir la température du milieu aux environs de 35° C. Le milieu réactionnel demeure limpide pendant environ la moitié de l'addition de soude, puis une cristallisation brusque se produit, accompagnée d'un échauffement. La température est contrôlée pour ne pas dépasser 35° C. L'agitation est maintenue encore 15 minutes; le milieu réactionnel a l'aspect d'une pâte blanche visqueuse.

Préparation de la solution cathodique.

On refroidit le réacteur au bain de glace jusqu'à une température inférieure ou égale à 10° C.

On ajoute alors 1,1 mole d'acide chlorhydrique, sous forme d'une solution aqueuse à 33%, assez lentement pour que la température reste inférieure à environ 10° C. Cette addition, menée sous forte agitation, est réalisée en 20 minutes environ. Au fur et à mesure de l'acidification, le milieu réactionnel devient de plus en plus fluide et prend finalement l'aspect d'une solution aqueuse homogène jaune clair.

Réduction électrochimique.

La solution obtenue est introduite dans le compartiment cathodique d'une cellule électrochimique.

La cellule utilisée est du type filtre-presse à trois compartiments : un compartiment cathodique entouré de deux compartiments anodiques séparés par deux diaphragmes constitués d'une membrane échangeuse de cations connue sous la dénomination "IONAC 3470".

Les anodes sont en $IrO_2$ sur titane connu sous le nom "DSA A-$O_2$-pH <2", et l'anolyte est une solution aqueuse sulfurique à 15-20% environ.

La cathode est en cuivre, sa surface utile est de 0,96 $dm^2$.

Avant la première opération, on ajoute au catholyte 7 mmoles de $Zn^{++}$ sous forme de sel minéral soluble. Pendant la première partie de la réduction correspondant environ à une quantité efficace d'électricité de 4 F/mole de composé nitro, $Zn^{++}$ reste en solution et ne participe pas à la réduction. Au fur et à mesure de l'élévation du potentiel de cathode, $Zn^{++}$ sera réduit et du zinc se dépose sur la cathode.

Cette cathode recouverte de Zn peut être utilisée directement sur une autre charge de composé nitro sans ajout complémentaire de $Zn^{++}$. En effet, pendant la première phase de cette deuxième opération, la couche de Zn est dissoute en $Zn^{++}$, et dans la seconde phase de la réduction électrochimique, elle se reforme. On peut ainsi enchaîner un grand nombre d'opérations de réduction sur la même cathode Cu-Zn sans traitement particulier, mis à part l'ajout de $Zn^{++}$ nécessaire pour compenser les pertes. La quantité de Zn entraînée dans le catholyte à chaque opération reste inférieure à 5% de la quantité initiale.

La température de la solution cathodique est maintenue entre 20 et 30° C pendant une première phase, jusqu'à ce que la quantité d'électricité réellement utilisée soit d'environ 4 F/mole de composé nitro.

Dans une seconde phase, on élève la température à 60° C. La solution se décolore progressivement.

Le catholyte est une solution chlorhydrique contenant 1,33 mole/kg de composé nitro. La densité moyenne de courant cathodique est de 18 A $dm^{-2}$. La tension interpolaire est de 4,6 V, pour une distance interpolaire de 0,5 cm.

Pour assurer à la fois l'agitation de la cellule, son dégazage, et son conditionnement thermique, on associe à l'appareil de réduction électrochimique un dispositif de recirculation externe du catholyte.

L'avancement de la réaction est contrôlé par dosage potentiométrique. On mesure la quantité de protons libres et de protons liés à la fonction hydroxylamine et à la fonction amine. La réduction est arrêtée

quand on constate la disparition de la fonction hydroxylamine.

On recueille alors la solution cathodique et on établit le bilan par dosage de la fonction amine dans le milieu et à partir de la quantité d'électricité réellement utilisée.

Est obtenue une solution contenant 0,74 mole d'amine/kg. La dilution en cours d'électrolyse est essentiellement due à l'eau de solvatation qui accompagne les protons qui traversent le diaphragme de l'anolyte vers le catholyte, assurant le passage du courant.

Le bilan montre que le rendement chimique est de 90% par rapport au nitrométhane consommé dans la formylation, et que l'efficacité du courant est de 50%, soit un consommation d'énergie (mesurée aux bornes de la cellule) de 15 kwh/kg.

Récupération du chlorhydrate ou de l'amino-2-propanediol-1,3.

On neutralise par NaOH l'acide en excès, puis on évapore à sec la solution aqueuse. Le résidu est repris par un alcool à bas poids moléculaire, méthanol ou éthanol, par exemple, ce qui permet d'éliminer le chlorure de sodium résiduel par filtration.

L'addition au filtrat d'un solvant chloré ou d'un ester de faible poids moléculaire, ($CH_2Cl_2$, $CHCl_3$, ou acétate d'éthyle, par exemple), permet de précipiter directement le chlorhydrate d'amino-2-propanediol-1,3. Ce produit est de très bonne pureté.

Pour obtenir l'amino-2-propanediol-1,3 libre, quand la réduction est terminée, on neutralise les protons en excès et ceux liés à la fonction amine, sous forme de $-NH_3^+$. On évapore alors presque à sec et on reprend par un alcool à bas poids moléculaire, on filtre le sel de sodium en excès, et, après élimination du solvant, on récupère l'amino-2-propanediol-1,3 par distillation sous pression réduite.

EXEMPLE 2

CHLORHYDRATE DE SERINOL

Formylation du nitrométhane.

Dans un réacteur cylindrique (diamètre 80) en verre à double enveloppe, équipé d'un agitateur à turbine inox (diamètre 40), à vitesse variable de 0 à 2000 t/mn, refroidi par circulation dans la double enveloppe d'eau glycolée à - 10 ° C, on prépare un pied de cuve constitué de :

- 50 g d'eau
- 1,8 g d'une solution aqueuse de soude à 50%
- 92 g de paraformaldéhyde (HCHO)3 soit 3,06 moles de formaldéhyde.

Après quelques minutes d'agitation, à l'aide d'une ampoule prolongée d'un tube d'introduction arrivant à 3 mm de la turbine, on introduit successivement :

- 96 g de $CH_3NO_2$ à 99,3% (1,53 moles)
  On joue sur la vitesse d'introduction et la vitesse de circulation de saumure pour maintenir une température de 30-35 ° C dans le mélange en réaction; l'exothermie très vive au début de l'addition devient beaucoup plus faible à la fin; l'opération dure environ 15 minutes; la solution obtenue est visqueuse;
- 128,5 g de NaOH à 50% (soit 1,606 moles au total : excès de 5%); la réaction peu exothermique au début, le devient brutalement au moment de la recristallisation qu'on provoque par amorçage quand environ 1/3 de la soude est ajoutée; on utilise la plus grande vitesse d'agitation afin d'éviter la prise en masse; on maintient la température à 30 ° C environ.

L'addition dure 30 minutes; on laisse la réaction se poursuivre encore pendant 15 minutes, en refroidissant. On obtient une pâte visqueuse.

Préparation de la solution de nitro-2-propanediol-1,3.

Dans un deuxième réacteur muni d'un agitateur identique, d'un dispositif d'addition d'acide à quelques mm de la turbine et d'une électrode de verre, on place 100 g d'eau que l'on refroidit vers 0 ° C puis on ajoute simultanément la bouillie de nitronate et la solution d'acide chlorhydrique de telle façon que le mélange reste dans une zone de pH comprise entre 6 et 8 et à une température voisine de 0 ° C. Cette neutralisation dure une heure.

Cette solution peut être, soit électrolysée tout de suite, soit conservée une quinzaine d'heures dans un réfrigérateur.

Electrolyse.

Après avoir ajouté à la solution froide une quantité d'HCl totale, telle que :

mmoles $H^+$ total = mmoles NaOH + (mmoles $CH_3NO_2$) x 1,1

on la transfère dans le circuit cathodique d'un appareillage électrochimique conforme à la description ci-dessus.

La cellule est un appareil connu sous le nom "Electrocell SU" du type "filtre presse" muni :

- d'une cathode de cuivre de 8 $dm^2$ de surface utile,
- de deux anodes en titane platiné (épaisseur Pt : 2,5 microns) de part et d'autre de la cathode,
- de deux diaphragmes en membrane échangeuse de cations, connus sous le nom "IONAC 3470".

Le catholyte est agité par recirculation dans un circuit comprenant le compartiment cathodique, un bac tampon en verre, un échangeur en verre, une pompe péristaltique.

L'anolyte, qui est une solution d'$H_2SO_4$, est agité et conditionné thermiquement au moyen d'un dispositif analogue.

Le débit de circulation du catholyte est tel que la vitesse de passage dans la cellule est supérieure à 2 $cm.s^{-1}$.

On ajoute au début de l'opération une quantité totale de zinc de 35 mmoles sous forme de $ZnCl_2$.

A l'aide d'une source de courant continu, on réalise entre les électrodes un champ électrique tel que la densité de courant sur la cathode soit de 24 $A.dm^{-2}$; cette intensité va décroitre progressivement jusqu'à 13,1 $A.dm^{-2}$, la densité de courant moyenne étant de 17,7 $A.dm^{-2}$; la tension interpolaire moyenne correspondante est de 4,8 volts.

On prélève périodiquement de petits échantillons du catholyte et on observe leurs réactions avec la liqueur de Fehling, tant qu'il y a de l'hydroxylaminopropanediol dans le milieu réactionnel, on voit apparaître la couleur brune caractéristique; quand le réactif conserve sa couleur bleue initiale, la réaction est terminée.

Après soutirage et pesée du catholyte, l'analyse potentiométrique permet d'établir le bilan de l'opération.

La température du catholyte a été maintenue à 30° C en début d'opération tant qu'il reste du dérivé nitré non réduit, puis portée à 60° C.

La concentration initiale en dérivé nitré est de 1,05 $moles.kg^{-1}$ et le rapport $H^+/RX$ est égal à 1,1. (R = propanediol et X = $NO_2$, NHOH ou $NH_2$).

Le produit final a la composition suivante :

| | |
|---|---|
| amino-2-propanediol-1,3 | 0,77 $mole.kg^{-1}$ |
| HCl | 0,89 $mole.kg^{-1}$ |
| hydroxylaminopropanediol | non décelable |

Le bilan montre que le rendement, en sérinol obtenu dans la solution par rapport à la nitroparaffine mise en oeuvre à la formylation, est de 85,5%.

Le rendement électrique est de 56%.

La consommation d'énergie est de 16,3 $kwh.kg^{-1}$.

La dilution de la solution cathodique en fin d'opération est causée par la solvatation des protons qui passent à travers le diaphragme et qui entraînent avec eux 22 g de $H_2O$ par Faraday.

Le traitement du catholyte selon les techniques d'extraction décrites ci-dessus permet d'obtenir à l'état très pur le chlorhydrate de sérinol.

EXEMPLE 3

SERINOL

Electrolyse.

On utilise un élément de cellule industrielle de type "filtre presse" connu sous le nom "ELECTRO PROD CELL", constituée d'une cathode en cuivre (dimension utile 0,4 $m^2$ : 0,8 x 0,5 m) et d'une anode DSA ($O_2$ pH <2) de même dimension séparées par un diaphragme en membrane échangeuse de cations, "IONAC MC 3470".

La distance entre diaphragme et électrode est de 2 mm. De part et d'autre de la membrane sont disposées des grilles en matière plastique qui jouent le rôle de promoteurs de turbulence.

Le compartiment cathodique est placé dans un circuit liquide comprenant une pompe de circulation, un bac de stockage et un échangeur thermique; la pompe assure une recirculation du catholyte à un débit tel que sa vitesse apparente dans le compartiment soit supérieure à 2 $cm.s^{-1}$; le compartiment anodique est placé sur un circuit analogue comprenant en outre une régulation de niveau par addition d'eau et un dispositif de mise en légère surpression par rapport au compartiment cathodique qui est à la pression atmosphérique; à cet effet, on fait barboter l'oxygène collecté sur l'anode dans un récipient contenant une certaine hauteur d'eau (15 cm).

La charge est introduite dans le compartiment cathodique et acidifiée pour que le rapport soit compris entre 1 et 1,15; elle correspond à la mise en oeuvre à la formylation de 35,8 moles de nitrométhane (soit 2,6 moles/kg); elle contient un résidu de $Na^+$ de 6,1 g/kg et on lui ajoute $Zn^{++}$ sous forme de $ZnSO_4.7H_2O$ à raison de 10 mmoles $Zn^{++}$ par kg de solution.

L'anolyte est une solution d'$H_2SO_4$ à 17%.

On établit un champ électrique continu, entre les deux électrodes, tel que, au départ, la densité de courant cathodique soit égale à environ 40 $A.dm^{-2}$ et que la tension interpolaire ne dépasse pas 5,5 volts; on obtient globalement une densité moyenne du courant cathodique de 27 $A.dm^{-2}$.

La température du catholyte est de 60° C.

On prélève périodiquement de petits échantillons du catholyte sur lesquels on effectue une analyse potentiométrique qui donne les teneurs en acidité libre, fonctions hydroxylamine et amine.

On arrête l'opération quand la teneur en R-NHOH est inférieure à 0,05 moles/kg.

L'analyse de la solution soutirée donne :

| | |
|---|---|
| $R-NH_2$ : | 1,19 $moles.kg^{-1}$ (amino-2-propanediol-1,3) |
| R-NHOH : | 0,05 $moles.kg^{-1}$ |
| $H^+$ : | 1,55 $moles.kg^{-1}$ |

correspondant à un rendement électrique de 60% et un rendement chimique de 84% par rapport au nitrométhane mis en oeuvre à la formylation. La consommation d'énergie est de 13,5 $kwh.kg^{-1}$.

La teneur en $Zn^{++}$ de la solution est de l'ordre de 10 ppm.

La dilution du catholyte entraînée par le passage d'eau à travers le diaphragme dû, notamment, à la solvatation des protons, est de 33 g d'eau par Faraday consommé.

## Purification par électro-électrodialyse.

Dans le même élément de cellule muni d'un diaphragme échangeur d'anions connu sous le nom de "IONAC MA 3475" et d'une cathode de cuivre neuve, on traite à la cathode une solution provenant de l'électrolyse décrite ci-dessus. Le circuit anodique est modifié de façon telle que la teneur en $H_2SO_4$ du compartiment anodique soit maintenue entre 3 et 4% par addition d'eau en circuit ouvert.

La solution cathodique sortant de l'électrolyseur est placée dans le compartiment cathodique de cette cellule, l'anolyte étant constitué d'une solution diluée d'acide sulfurique et maintenue à cette concentration par addition d'eau (circuit ouvert); quand on crée un champ électrique entre les deux électrodes, les ions $SO_4$ sont transférés vers le compartiment anodique et l'opération est poursuivie jusqu'à élimination presque totale de ces ions du compartiment cathodique; néanmoins, à cause de la présence d'ions $Na^+$ en concentration faible, mais non nulle, on doit l'arrêter quand la concentration en ions $SO_4$ = est stoechiométriquement équivalente à celle des ions sodium.

La température des électrolytes est de 60° C. On fait passer un courant continu entre les électrodes en maintenant une tension interpolaire de 9,2 volts; la densité moyenne de courant sur le diaphragme est de 13 $A.dm^{-2}$ (maximum 20 $A.dm^{-2}$). On suit l'évolution de l'opération par mesure du pH du catholyte; on l'arrête quand celui-ci est très alcalin ( 10).

Le tableau ci-dessous donne les compositions initiales et finales du catholyte.

| | Initial | Final |
|---|---|---|
| $R-NH_2$ mole.kg$^{-1}$ | 1,13 | 1,3 |
| R-NHOH mole.kg$^{-1}$ | 0,03 | 0 |
| $H^+$ total mole.kg$^{-1}$ | 1,4 | 0 |
| $Na^+$ g.kg$^{-1}$ | | 3 |

$R-NH_2$ = amino-2-propanediol-1,3
R-NHOH = hydroxyamino-2-propanediol-1,3
$H^+$ total = acidité sulfurique libre + acidité liée sous forme de $(R-^+NH_3)_2SO_4$ et $(R-^+NH_2OH)_2SO_4$

La solution acide de sulfate d'amine est donc transformée en une solution aqueuse d'amino-alcool contenant 9,2 g.kg$^{-1}$ de $Na_2SO_4$; la réduction s'est poursuivie aux dépens des traces de fonction hydroxylamino restante et, du fait de la solvatation des ions $SO_4$ =, la solution de sérinol a été un peu concentrée.

Le rendement chimique de l'opération est de 97,7% (poids initial d'amino-alcool/poids final d'amino-alcool).

Par évaporation de l'eau sous pression réduite, (20 à 50 millibar) puis évaporation en couche mince sous vide poussé (1 millibar), on obtient du sérinol de pureté supérieure à 99%.

Le rendement électrique - défini comme le rapport entre la quantité d'électricité théoriquement nécessaire au transfert de tous les ions $SO_4$ à travers le diaphragme et la quantité d'électricité réellement utilisée - est de 79,4%. la consommation d'énergie est de 4,7 kwh.kg$^{-1}$.

Le rendement chimique global de l'ensemble des opérations formylation (électrolyse-électro-électrodialyse) est donc de 82% par rapport au nitrométhane mis en oeuvre dans la formylation.

Des rendements analogues ont été obtenus en utilisant pour l'électro-électrodialyse une membrane perméable aux anions connu sous le nom de "NEOSEPTA ACL E 5P" à la place de "IONAC MA 3475".

## Revendications

1. Procédé de préparation de l'amino-2-propane diol-1,3 ou de ses sels, dans lequel on réalise au moins les étapes suivantes :
   a) on réalise une formylation par réaction du formaldéhyde et du nitrométhane en milieu aqueux alcalin;
   b) on acidifie la solution obtenue;
   c) on réalise l'électroréduction cathodique de la solution acidifiée; et
   d) on sépare l'amino-2-propanediol-1,3 ou son sel de la solution cathodique.

2. Procédé selon la revendication 1, caractérisé en ce que dans le milieu alcalin, la concentration molaire en $OH^-$ est 20% supérieure à la concentration stoechiométrique, de préférence environ 5% supérieure.

3. Procédé selon la revendication 1 ou 2, caractérisé en ce qu'à l'étape a), la réaction a lieu à une température inférieure à 50 °C, de préférence inférieure à environ 35 °C.

4. Procédé selon l'une des revendications 1 à 3, caractérisé en ce qu'à l'étape b), l'acidification est réalisée par apport d'acide méthane-sulfonique, benzène-sulfonique, toluène-sulfonique, chlorhydrique, bromhydrique, fluorhydrique, nitrique, sulfurique ou phosphorique, de préférence par apport d'acide sulfurique ou chlorhydrique en solution aqueuse.

5. Procédé selon l'une des revendications 1 à 4, caractérisé en ce qu'à l'étape b), la température est maintenue inférieure à la température ambiante, et de préférence inférieure à environ 10 °C.

6. Procédé selon l'une des revendications 1 à 5, caractérisé en ce qu'à l'étape b), l'acidification a lieu sous agitation.

7. Procédé selon l'une des revendications 1 à 6, caractérisé en ce que durant l'étape b), le pH est

maintenu entre 5 et 7.

**8.** Procédé selon l'une des revendications 1 à 7, caractérisé en ce qu'à l'étape c), la concentration initiale d'acide est telle que $H^+/C_3H_7O_2\text{-}NO_2$ est compris entre 1 et 1,5, de préférence égal à environ 1,3.

**9.** Procédé selon l'une des revendications 1 à 8, caractérisé en ce qu'à l'étape c), la température du catholyte est maintenue inférieure à une température de 35 °C, de préférence inférieure à une température d'environ 30 °C, et dans une seconde phase, la température du catholyte est maintenue à une température supérieure à 50 °C, et de préférence supérieure ou égale à 60 °C.

**10.** Procédé selon l'une des revendications 1 à 9, caractérisé en ce qu'à l'étape c), pour réaliser l'électro-réduction cathodique, on utilise une cathode en métal-support choisie parmi le cuivre, le nickel, le plomb et l'acier inoxydable, recouverte d'un élément actif choisi parmi le zinc, le cadmium et l'étain.

**11.** Procédé selon la revendication 10, caractérisé en ce que le métal-support est le cuivre.

**12.** Procédé selon l'une des revendications 10 ou 11, caractérisé en ce que l'élément actif est le zinc.

**13.** Procédé selon l'une des revendications 1 à 12, caractérisé en ce qu'à l'étape b), après l'acidification de la solution obtenue à l'étape a), on sépare le sel de neutralisation.

**14.** Procédé selon l'une des revendications 1 à 13, caractérisé en ce qu'à la fin de l'étape c), on purifie le catholyte par électro-électrodialyse.

Office européen des brevets

# RAPPORT DE RECHERCHE EUROPEENNE

Numéro de la demande

**EP 90 40 3405**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | FR-A-2 614 044 (L'AIR LIQUIDE)<br>* Revendications *<br>– – – | 1-14 | C 07 C 213/02<br>C 07 C 215/10 |
| A,D | EP-A-0 071 037 (DYNAMIT NOBEL AG)<br>* Revendications *<br>– – – – – | 1 | |

DOMAINES TECHNIQUES RECHERCHES (Int. Cl.5)

C 07 C 213/00

Le présent rapport de recherche a été établi pour toutes les revendications

| Lieu de la recherche | Date d'achèvement de la recherche | Examinateur |
|---|---|---|
| La Haye | 25 mars 91 | SANCHEZ Y GARCIA J.M |

CATEGORIE DES DOCUMENTS CITES

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention

E: document de brevet antérieur, mais publié à la date de dépôt ou après cette date
D: cité dans la demande
L: cité pour d'autres raisons

&: membre de la même famille, document correspondant